# EUROPEAN PATENT APPLICATION

(11) **EP 4 555 913 A1**
(43) Date of publication of application: **21.05.2025**
(21) Application number: 23839951.3
(22) Date of filing: 12.07.2023
(51) Int. Cl.: A61B 1/00, A61B 34/30, A61B 46/10

(54) **ENDOSCOPE DRIVING ASSEMBLY**

(30) Priority: 13.07.2022 KR 20220086570; 07.07.2023 KR 20230088668
(71) Applicant: ROEN Surgical, Inc., Daejeon 34051 (KR)
(72) Inventor: YANG, Unje, Daejeon 34141 (KR); LEE, Daesung, Daejeon 34141 (KR); PARK, Jinyong, Daejeon 34141 (KR)
(74) Representative: Stolmár & Partner Patentanwälte PartG mbB
(86) International application number: PCT/KR2023/009915
(87) International publication number: WO 2024/014859

(57) **Abstract**

An endoscope driving assembly for supporting and driving an endoscopic device disposed at a slave device according to an embodiment may comprise: a coupler for supporting the endoscopic device; and a mount to which the coupler is mounted and which receives a signal from a master device to control the endoscopic device, wherein the coupler is replaceably provided to support a plurality of endoscopic devices having different shapes.

## Description

### Technical Field

The present disclosure relates to an endoscope driving assembly.

### Background Art

An endoscope is a medical device that allows direct viewing of internal organs or body cavities, and is a device designed to insert a medical instrument into and observe an organ where a lesion cannot be directly seen without surgery or autopsy. Types of endoscopes include, for example, bronchoscopes, esophagoscopes, gastroscopes, duodenoscopes, proctoscopes, cystoscopes, laparoscopes, or ureteroscopes.

Meanwhile, since the specifications of endoscopes manufactured by various manufacturers are different, there is a need for an endoscopic surgical system to mount each individual endoscope with different specifications on a slave device.

### Disclosure

### Technical Solution

According to an embodiment, an endoscope driving assembly for supporting and driving an endoscopic device disposed on a slave device is provided. The endoscope driving assembly includes: a coupler configured to support the endoscopic device; and a mount on which the coupler is mounted and configured to receive a signal from a master device to control the endoscopic device, wherein the coupler may be replaceably provided to support a plurality of endoscopic devices with different shapes.

According to an embodiment, the coupler may include: a coupler body capable of accommodating at least a portion of the endoscopic device; and a main hole formed through the coupler body and configured to accommodate a handle of the endoscopic device.

According to an embodiment, the mount may include: a mount body configured to fasten to the coupler body; a rotor disposed on the mount body and capable of rotating in any one direction by receiving a signal from the master device; and a handle holder connected to the rotor and configured to rotate the handle of the endoscopic device with the handle being accommodated in the main hole of the coupler.

According to an embodiment, the handle holder may be replaceable.

According to an embodiment, the coupler may further include a first detection target pressed by the endoscopic device and configured to move in a direction toward the mount body in a state where the mount body and the coupler body are fastened together.

According to an embodiment, the coupler may further include a second detection target disposed on a side of the coupler body facing the mount body.

According to an embodiment, the mount may further include: a first sensor configured to detect the first detection target moved in the direction toward the mount body; and a second sensor configured to detect the second detection target while the coupler body is fastened to the mount body.

According to an embodiment, the mount may further include: a vertical extension part provided around a rotation axis of the rotor; and guide grooves provided spaced apart from each other on an outer peripheral surface of the vertical extension part.

According to an embodiment, the coupler may further include guide protrusions respectively accommodated in the guide grooves, wherein with the guide protrusions accommodated in the guide grooves, the coupler may rotate in one direction around the rotation axis of the rotor and may be fastened to the mount.

According to an embodiment, the mount may further include a hook provided so as to be caught on the coupler while the mount is fastened to the coupler.

According to an embodiment, the hook may include: a hook body supported by the mount body; and a hook head configured to protrude from the hook body toward the coupler body.

According to an embodiment, the coupler may further include an elastic protrusion disposed on a side of the coupler body facing the hook, wherein with the coupler body caught on the hook head, the elastic protrusion may press the hook body.

According to an embodiment, a method for using an endoscope driving assembly for supporting and driving an endoscopic device disposed on a slave device is provided, wherein the endoscope driving assembly may include: a coupler configured to support the endoscopic device; and a mount on which the coupler is mounted and configured to receive a signal from a master device to control the endoscopic device, wherein the coupler may be replaceably provided to support a plurality of endoscopic devices with different shapes.

### Description of Drawings

FIG. 1 is a view showing an endoscopic surgical system according to an embodiment.
FIG. 2 is a perspective view showing a driving part of a slave device according to an embodiment.
FIG. 3 is a perspective view showing an endoscopic device, a coupler, and a mount manufactured by Boston Scientific, according to an embodiment.
FIG. 4 is a perspective view showing a coupler according to an embodiment.
FIG. 5 is a perspective view showing a coupler supporting an endoscopic device manufactured by Karl Storz according to an embodiment.
FIG. 6 is a perspective view showing a coupler supporting an endoscopic device manufactured by OTU Medical according to an embodiment.

### Best Mode

Specific structural or functional descriptions of embodiments of the present disclosure are disclosed for illustrative purposes only and may be changed and implemented in various forms. Therefore, the actual implementation form is not limited to the specific disclosed embodiments, and the scope of the present specification includes changes, equivalents, or substitutes included in the technical idea described in the embodiments.

Terms such as first or second may be used to describe various components, but these terms should be interpreted only for the purpose of distinguishing one component from another. For example, a first component may be named a second component, and similarly the second component may also be named a first component.

It should be understood that when a component is said to be "connected" to another component, this includes not only the case of being directly connected but also indirectly connected with another component in between.

The singular expression may include a plural expression unless the context clearly indicates otherwise. In addition, it should be understood that in the present specification, terms such as "comprise" or "have" are intended to designate that a feature, number, step, operation, component, part, or combination thereof described in the specification exists, and do not preclude the possibility of addition or existence of one or more other features or numbers, steps, operations, components, parts, or combinations thereof.

Components included in one embodiment and components including common functions will be described using the same names in other embodiments. Unless stated to the contrary, the description given in one embodiment may be applied to other embodiments, and detailed description will be omitted to the extent of redundancy.

Unless otherwise defined, all terms used herein, including technical or scientific terms, have the same meaning as commonly understood by a person skilled in the art. Terms such as those defined in the commonly used dictionaries should be construed as having meanings consistent with the meanings in the context of the related art and shall not be construed in ideal or excessively formal meanings unless expressly defined in this application.

Hereinafter, the embodiments will be described in detail with reference to the attached drawings. In describing with reference to the attached drawings, identical components will be assigned the same reference numerals regardless of the reference numerals in the drawings, and redundant descriptions thereof will be omitted.

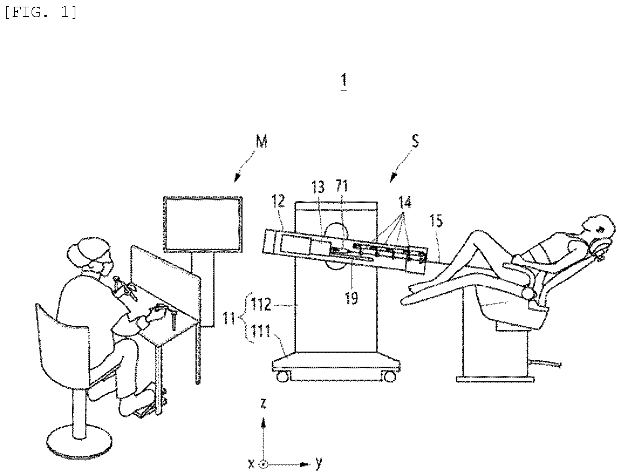

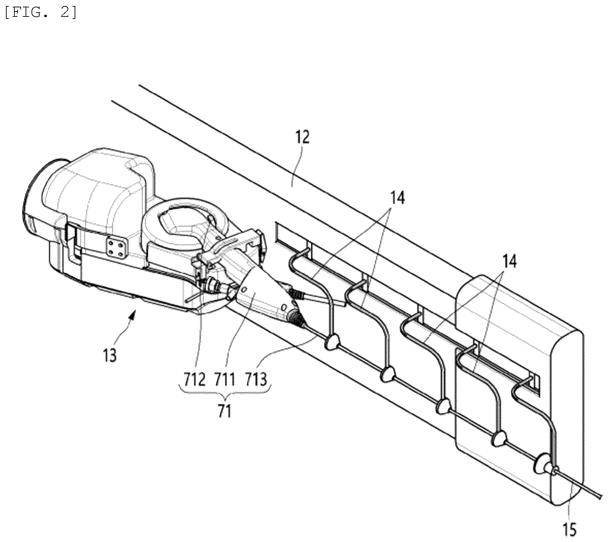

FIG. 1 is a view showing an endoscopic surgical system according to an embodiment, and FIG. 2 is a perspective view showing a driving part of a slave device according to an embodiment.

Referring to FIGS. 1 and 2, an operator (surgeon) according to an embodiment may perform surgery using an endoscopic surgical system 1. The endoscopic surgical system 1 may include a master device M and a slave device S.

The master device M may be controlled by the operator. For example, the master device M may include a screen and a controller. The operator uses the controller to control the slave device S connected to the master device M and may check the progress of a surgery in real time through the screen. The master device M and the slave device S may be connected wired or wirelessly. The operator may directly enter commands on the screen to control the slave device S. For example, the operator may input values such as the height and rotation angle of the slave device S on the screen. Meanwhile, unlike what is shown in the drawings of the present specification, the screen and controller of the master device M may be provided as an integrated unit.

The slave device S may receive signals from the master device M and perform actions necessary for surgery. The slave device S may include a surgical instrument that is inserted into the patient's body. For example, the slave device S may include an endoscopic device, a laser for crushing kidney stones, a basket for holding the crushed kidney stones, etc. The slave device S may include a support part 11, a driving part 12, an endoscope driving assembly 13, an endoscope holder 14, an access sheath 15, and an endoscopic device 71.

The support part 11 may be provided to be movable with respect to the ground. The support part 11 may include a support base 111 to which at least one wheel is attached, and a support body 112 connected to the support base 111. The support body 112 may move relative to the support base 111 by receiving a signal from the master device M. For example, the support body 112 may rotate about the z-axis as a rotation axis or move translationally in the x-axis and y-axis directions with respect to the support base 111.

The driving part 12 is supported by the support part 11 and may move relative to the support part 11. For example, the drive unit 12 may receive a signal from the master device M and rotate the x-axis as a rotation axis with respect to the support body 112, or translate in the z-axis direction.

The endoscope driving assembly 13 is connected to a rail 19 provided on the driving part 12 and may be provided to be slidable in the longitudinal direction of the driving part 12. The endoscope driving assembly 13 supports the endoscopic device 71 and may control the endoscopic device 71 by receiving signals from the master device M. For example, the endoscope driving assembly 13 may move a handle 712 of the endoscopic device 71, which controls the direction in which an endoscope 713 of the endoscopic device 71 is bent. While the endoscopic device 71 is supported on the endoscope driving assembly 13, the relative movement of the endoscopic device 71 with respect to the endoscope driving assembly 13 may be limited. Errors arising from the movement of the endoscopic device 71 may be reduced, and the safety and accuracy of surgery may be improved.

The endoscope holder 14 may support the endoscope 713 of the endoscopic device 71. The endoscope holder 14 may prevent the endoscope 713 of the endoscopic device 71 from buckling when the endoscope driving assembly 13 slides or rotates relative to the driving part 12. For example, a plurality of endoscope holders 14 may be provided along the longitudinal direction of the endoscope 713 of the endoscopic device 71, and the endoscope holders 14 may be spaced apart from each other. For example, the endoscope holders 14 may be capable of sliding in one direction along the longitudinal direction of the driving part 12.

The access sheath 15 is connected to the end of the driving part 12 and may be inserted into the patient's body during surgery. The endoscope 713 of the endoscopic device 71 may pass through the access sheath 15 to reach the patient's surgical site.

The endoscopic device 71 mounted on the endoscope driving assembly 13 may be an endoscopic device that controls the bending of the endoscope 713 from the relative movement of the handle 712 with respect to a main body 711 of the endoscopic device 71. For example, when the handle 712 of the endoscopic device 71 is moved in one direction relative to the main body 711 of the endoscopic device 71, the endoscope 713 of the endoscopic device 71 may be bent to the left, whereas when the handle 712 of the endoscopic device 71 is moved in the opposite direction of one direction, the endoscope 713 of the endoscopic device 71 may be bent to the right.

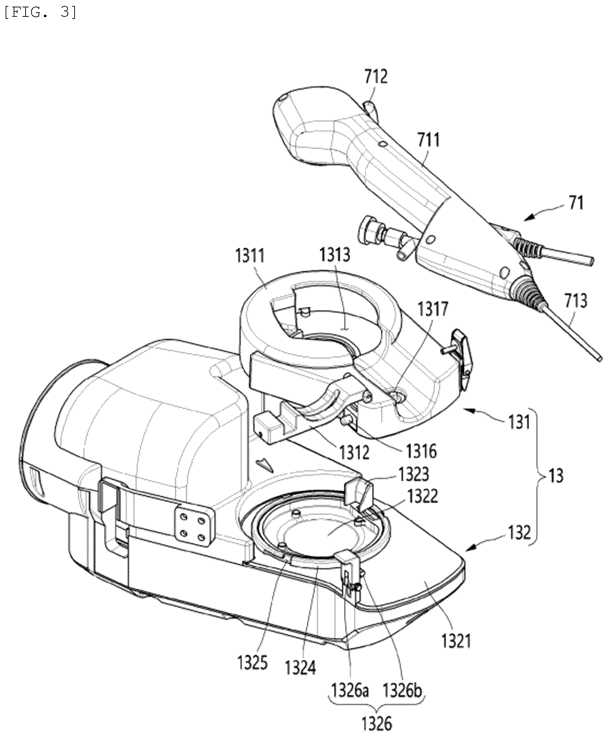

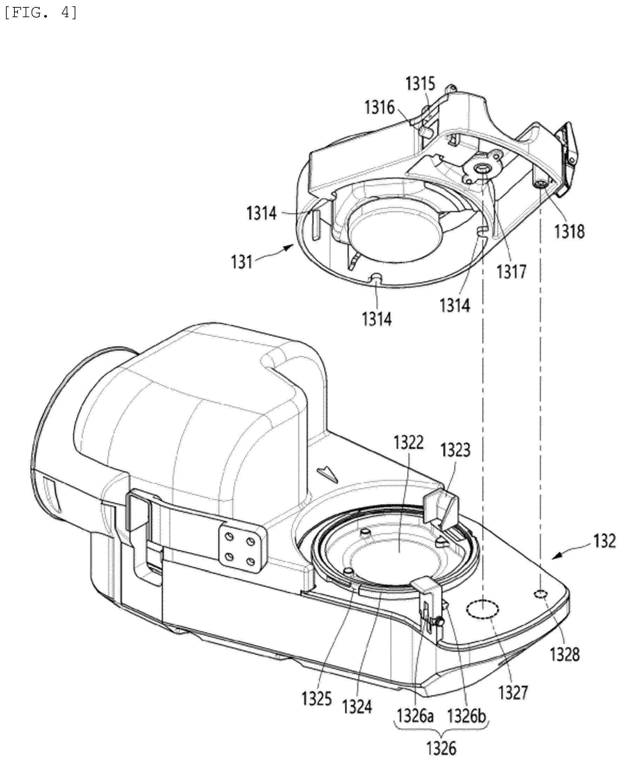

FIG. 3 is a perspective view showing an endoscopic device, a coupler, and a mount manufactured by Boston Scientific, according to an embodiment, and FIG. 4 is a perspective view showing a coupler according to an embodiment.

Referring to FIGS. 3 and 4, the endoscope driving assembly 13 according to an embodiment may include a coupler 131 and a mount 132. The coupler 131 may support the endoscopic device 71 and secure a portion of a drape (not shown) to the mount 132. In this case, the drape refers to the vinyl that, within an endoscopic surgical system, separates the master and slave devices from microorganisms and other contaminating agents. After one end of the drape is placed between the coupler 131 and the mount 132, the coupler 131 is mounted on the mount 132, and a portion of the drape is pressed by the coupler 131 and the mount 132. Due to this arrangement, a portion of the drape may be secured to the endoscope driving assembly 13 and the endoscope driving assembly 13 may be completely covered by the remaining portion of the drape. The coupler 131 may be a disposable component that is replaced after surgery is completed for hygiene reasons.

The coupler 131 may have a variety of shapes to accommodate endoscopic devices 71 manufactured by several manufacturers, including Boston Scientific, Karl Storz, and OTU Medical. The specifications of the endoscopic device 71 manufactured by various manufacturers may be different. For example, the position of the handle 712 with respect to the main body 711 of the endoscopic device 71, the direction in which the endoscope 713 extends from the main body 711, etc. may be different. When using the endoscope driving assembly 13 having a predetermined shape, it may be difficult to use endoscopic devices 71 having various specifications interchangeably. The operator may use various types of endoscopic device 71 by replacing the coupler 131 with a different shape for the mount 132 with a predetermined shape. Hereinafter, for convenience of explanation, the coupler 131 that accommodates the endoscopic device 71 manufactured by Boston Scientific will be described. The coupler 131 may include a coupler body 1311, a cover 1312, a main hole 1313, and a guide protrusion 1314.

The endoscopic device 71 may be disposed on the coupler body 1311. The coupler body 1311 may include a groove recessed along the edge of the endoscopic device 71 to accommodate the endoscopic device 71. When the endoscope device 71 is accommodated in the coupler body 1311, rotation of the endoscopic device 71 with respect to the coupler body 1311 may be limited.

The cover 1312 has one end hingedly connected to the coupler body 1311 and may prevent the endoscopic device 71 from being separated from the coupler body 1311. After the endoscopic device 71 is accommodated in the coupler body 1311, the other end of the cover 1312 may be fixed to the coupler body 1311. In this state, the endoscopic device 71 is pressed by the cover 1312, and the relative movement of the cover 1312 with respect to the coupler body 1311 may be limited.

The main hole 1313 may be formed through the coupler body 1311. The handle 712 of the endoscopic device 71 may be accommodated in the main hole 1313. The main hole 1313 may be formed around one axis and along the circumference thereof. The main hole 1313 may be a space in which the handle 712 may move relative to the main body 711 of the endoscopic device 71. Since the main body 711 of the endoscopic device 71 is fixed by the coupler body 1311 and the cover 1312, the endoscope 713 of the endoscopic device 71, the bending of the endoscope 713 of the endoscopic device 71 may be controlled by moving the handle 712 relative to the main body 711.

The mount 132 may include a mount body 1321, a rotor 1322, a handle holder 1323, a vertical extension part 1324, and a plurality of guide grooves 1325.

The mount body 1321 may be connected to the rail provided on the driving part. The rotor 1322 that is capable of rotating about one axis may be provided on one side of the mount body 1321. The rotor 1322 may rotate clockwise and counterclockwise by receiving signals from the master device.

The handle holder 1323 may be connected to the rotor 1322 and connected to the handle 712 of the endoscopic device 71. For example, the handle holder 1323 may be hingedly connected to the edge of the rotor 1322. The handle 712 of the endoscopic device 71 may be positioned between a pair of pressing surfaces of the handle holder 1323. The pair of opposing pressing surfaces may press one side and the other side of the handle 712 of the endoscopic device 71. For example, when the rotor 1322 rotates clockwise, one of the pressing surfaces presses one side of the handle 712 of the endoscopic device 71 and the handle 712 of the endoscopic device 71 may rotate clockwise. On the other hand, when the rotor 1322 rotates counterclockwise, the other of the pressing surfaces presses the other side of the handle 712 of the endoscopic device 71 and the handle 712 of the endoscopic device 71 may rotate counterclockwise.

In an embodiment, the handle holder 1323 may be provided to be replaceable. For example, the handle 712 of the endoscopic device 71 manufactured by various manufacturers may have different widths. Due to the gap that exists between the pressing surfaces of the handle holder 1323 and the handle 712 of the endoscopic device 71, when the handle holder 1323 in a stationary state rotates in one direction, a delay may occur while the handle 712 of the endoscopic device 71 is pressed by the handle holder 1323. By using the handle holder 1323 fixed to the individual handle 712 having different widths, errors resulting from delay time may be reduced, and maintenance costs of the endoscope driving assembly 13 may be reduced.

The vertical extension part 1324 protrudes from the mount body 1321 and may be formed around the rotation axis of the rotor 1322. For example, the vertical extension part 1324 may be provided along the edge of the rotor 1322 and may prevent foreign substances from penetrating into the gap between the mount body 1321 and the rotor 1322. A plurality of guide grooves 1325 may be provided on the outer peripheral surface of the vertical extension part 1324.

The guide grooves 1325 may be formed to be spaced apart from each other around the rotation axis of the rotor 1322. Each of the guide grooves 1325 may be formed around the rotation axis of the rotor 1322. Dud to this structure, after the guide protrusions 1314 of the coupler 131 are inserted into the guide grooves 1325, the coupler 131 rotates along the direction in which the guide grooves 1325 are formed so as to be mounted on the mount 132. With the coupler 131 mounted on the mount 132, vertical movement of the coupler 131 with respect to the mount 132 may be limited.

In the drawings of the present specification, although the number of guide protrusions 1314 and guide grooves 1325 is shown as three each, it should be noted in advance that the number of guide protrusions 1314 and guide grooves 1325 is not limited thereto.

With the coupler 131 mounted on the mount 132, rotation of the coupler 131 with respect to the mount 132 may be limited by a hook 1326 and an elastic protrusion 1316.

The hook 1326 may include a hook body 1326a supported by the mount body 1321, and a hook head 1326b protruding from the hook body 1326a. With the coupler 131 mounted on the mount 132, the hook head 1326b may be received in a hook receiving groove 1315 formed on the coupler body 1311 facing the hook body 1326a and caught on the coupler body 1311. When the coupler body 1311 attempts to rotate in the direction in which the hook receiving groove 1315 moves away from the hook head 1326b, the coupler body 1311 is caught by the hook head 1326b, and thus the rotation of the coupler body 1311 may be limited.

The elastic protrusion 1316 protrudes from the coupler body 1311 and may be compressed when pressed, and may be returned to the original position thereof by elastic force when not pressed. With the hook head 1326b caught on the coupler body 1311, the elastic protrusion 1316 may be in a state of pressing the hook body 1326a. When the coupler body 1311 rotates in the direction in which the hook receiving groove 1315 approaches the hook head 1326b, the rotation of the coupler body 1311 may be limited due to the restoring force of the elastic protrusion 1316. The hook head 1326b may remain caught on the coupler body 1311. This state may referred to as a "fully engaged state".

The coupler 131 may further include at least one of a first detection target 1317 and a second detection target 1318. The operator may determine whether the endoscopic device 71 is supported by the coupler 131 by means of the first detection target 1317. The operator may check whether the coupler 131 is fixed to the mount 132 by means of the second detection target 1318.

The first detection target 1317 may be provided to penetrate the coupler body 1311 in a direction in which the endoscopic device 71 is accommodated in the coupler 131. The upper part of the first detection target 1317 may exist on a side of the coupler body 1311 where the endoscopic device 71 is disposed, whereas the lower part of the first detection target 1317 may exist on a side opposite to the side of the coupler body 1311 where the endoscopic device 71 is disposed. For example, the upper part of the first detection target 1317 may include an elastic material, and the lower part of the first detection target 1317 may include metal.

The upper part of the first detection target 1317 may be in a state of protruding from the coupler body 1311 when the endoscopic device 71 is not accommodated in the coupler 131. When the endoscopic device 71 is accommodated in the coupler 131 while the coupler 131 is mounted on the mount 132, the upper part of the first detection target 1317 is pressed by the endoscopic device 71, and the lower part of the first detection target 1317 may be moved toward the mount 132. A first sensor 1327 provided on the mount body 1321 may detect that the metal included on the lower part of the first detection target 1317 is within a preset distance and generate a signal. The first sensor 1327 may be placed inside the mount body 1321, for example. Meanwhile, when the first detection target 1317 is no longer pressed by the endoscopic device 71, the first detection target 1317 may return to the original position thereof by restoring force.

The second detection target 1318 may be disposed on a side of the coupler body 1311 facing the mount 132. The second detection target 1318 may be metal, for example. In a state where the coupler 131 is guided by the guide grooves 1325 and connected to the vertical extension part 1324, and the hook head 1326b of the mount 132 is fully engaged with the coupler body 1311, the second detection target 1318 may overlap a second sensor 1328 provided on the mount body 1321 based on the direction in which the endoscopic device 71 is accommodated in the coupler 131. The second sensor 1328 may detect that the second detection target 1318 is within a preset distance and generate a signal. The second sensor 1328 may be placed inside the mount body 1321, for example.

Meanwhile, when metal is used for both the first detection target 1317 and the second detection target 1318, the operator may appropriately set the detection ranges of the first sensor 1327 and the second sensor 1328, so that the first sensor 1327 does not detect the second detection target 1318 and the second sensor 1328 does not detect the first detection target 1317.

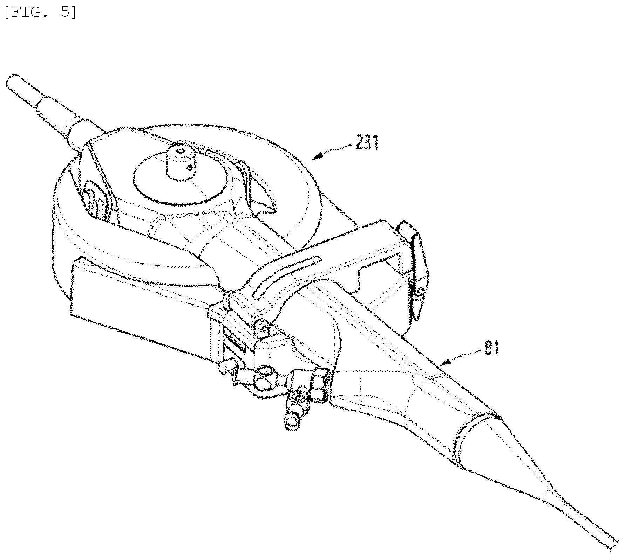

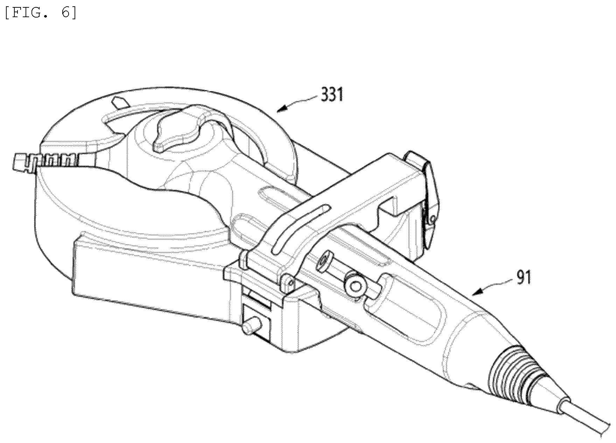

FIG. 5 is a perspective view showing a coupler supporting an endoscopic device manufactured by Karl Storz, and FIG. 6 is a perspective view showing a coupler supporting an endoscopic device manufactured by OTU Medical.

Referring to FIGS. 5 and 6, a coupler 231 supporting an endoscopic device 81 manufactured by Karl Storz according to an embodiment and a coupler 331 supporting an endoscopic device 91 manufactured by OTU Medical according to an embodiment.

A coupler (e.g., coupler 131 in FIG. 3, coupler 231 in FIG. 5, and coupler 331 in FIG. 6) may be designed considering not only the shape of an endoscopic device (e.g., endoscopic device 71 in FIG. 3, endoscopic device 81 in FIG. 5, and endoscopic device 91 in FIG. 6), but also the width of a handle of the endoscopic device 71, 81, or 91 and the driving position of the handle of the endoscopic device 71, 81, or 91. For example, depending on the shape of a main body of the endoscopic device 71, 81, or 91, the location and depth of a groove formed in a coupler body may be designed differently. For example, the location and width of a main hole formed in the coupler body may be designed differently depending on the length of the handle protruding from the main body and the range in which the handle moves.

When an endoscope is not bent, the initial position, which is the position of the handle relative to the main body, may be different. In this case, a rotor of a mount may be rotated in advance to move a handle holder to the initial position of the handle, and then the handle holder may be fixed to the handle. The lengths of the endoscopic devices 71, 81, and 91 manufactured by various manufacturers may be different. With the endoscopic device 71, 81, or 91 supported on the driving assembly, the distance between a main body of an endoscope and an endoscope holder placed closest to the main body among a plurality of endoscope holders (hereinafter referred to as a "first endoscope holder") may vary for each endoscopic device 71, 81, or 91.

As the angle between the direction in which the endoscope of the endoscopic device 71, 81, or 91 enters the first endoscope holder and the direction in which the endoscope holders are arranged in a row increases, buckling of the endoscope may increase. When the endoscope extending from the main body of the endoscopic device 71, 81, or 91 passes through the endoscope holders in a straight line without bending, buckling of the endoscope may be reduced. When buckling of the endoscope occurs, it is difficult for the endoscope to reach the patient's affected area, and errors may occur between the user's manipulation and the movement of the endoscope.

In order to reduce buckling of the endoscopic device 71, 81, or 91, the angle at which each endoscopic device 71, 81, or 91 is accommodated in the driving assembly may be different. In other words, when the endoscopic device 71, 81, or 91 is accommodated in the coupler 131, 231, or 331, and the coupler 131, 231, or 331 is fully engaged with the mount, the angle formed by the longitudinal direction of the endoscopic device 71, 81, or 91 and the longitudinal direction of the driving part may be different. For example, by designing the positions of guide protrusions formed on the coupler body to be different, the angle formed by the longitudinal direction of the endoscopic device 71, 81, or 91 and the longitudinal direction of the driving part may be adjusted.

Meanwhile, a modeling program may be used to determine the placement of the endoscopic device 71, 81, or 91 so that buckling of the endoscope is minimized. For example, by modeling the shape of each endoscopic device 71, 81, or 91 to calculate values such as the length of the main body, the angle of the endoscope protruding from the main body, and the distance between the main body and the first endoscope holder, the optimal placement position of the endoscopic device 71, 81, or 91 to minimize buckling may be determined.

Although the embodiments have been described with limited drawings as described above, those skilled in the art can apply various technical modifications and variations based on the above. For example, even if the techniques described are performed in a different order than the described method and/or components of the described system, structure, device, circuit, etc. are combined in a form other than the described method, or are replaced or substituted by other components or equivalents, appropriate results may be achieved.

Therefore, other implementations, other embodiments, and equivalents of the claims also fall within the scope of the appended claims.

## Claims

1. An endoscope driving assembly for supporting and driving an endoscopic device disposed on a slave device, the endoscope driving assembly comprising:
a coupler configured to support the endoscopic device; and
a mount on which the coupler is mounted and configured to receive a signal from a master device to control the endoscopic device,
wherein the coupler is replaceably provided to support a plurality of endoscopic devices with different shapes.

2. The endoscope driving assembly of claim 1, wherein the coupler comprises:
a coupler body capable of accommodating at least a portion of the endoscopic device; and
a main hole formed through the coupler body and configured to accommodate a handle of the endoscopic device.

3. The endoscope driving assembly of claim 2, wherein the mount comprises:
a mount body configured to fasten to the coupler body;
a rotor disposed on the mount body and capable of rotating in any one direction by receiving a signal from the master device; and
a handle holder connected to the rotor and configured to rotate the handle of the endoscopic device with the handle being accommodated in the main hole of the coupler.

4. The endoscope driving assembly of claim 3, wherein the handle holder is replaceable.

5. The endoscope driving assembly of claim 3, wherein the coupler further comprises:
a first detection target pressed by the endoscopic device and configured to move in a direction toward the mount body in a state where the mount body and the coupler body are fastened together.

6. The endoscope driving assembly of claim 5, wherein the coupler further comprises:
a second detection target disposed on a side of the coupler body facing the mount body.

7. The endoscope driving assembly of claim 6, wherein the mount further comprises:
a first sensor configured to detect the first detection target moved in the direction toward the mount body; and
a second sensor configured to detect the second detection target while the coupler body is fastened to the mount body.

8. The endoscope driving assembly of claim 3, wherein the mount further comprises:
a vertical extension part provided around a rotation axis of the rotor; and
guide grooves provided spaced apart from each other on an outer peripheral surface of the vertical extension part.

9. The endoscope driving assembly of claim 8, wherein the coupler further comprises guide protrusions respectively accommodated in the guide grooves,
wherein with the guide protrusions accommodated in the guide grooves, the coupler rotates in one direction around the rotation axis of the rotor and is fastened to the mount.

10. The endoscope driving assembly of claim 9, wherein the mount further comprises:
a hook provided so as to be caught on the coupler while the mount is fastened to the coupler.

11. The endoscope driving assembly of claim 10, wherein the hook comprises:
a hook body supported by the mount body; and
a hook head configured to protrude from the hook body toward the coupler body.

12. The endoscope driving assembly of claim 11, wherein the coupler further comprises an elastic protrusion disposed on a side of the coupler body facing the hook,
wherein with the coupler body caught on the hook head, the elastic protrusion presses the hook body.

13. A method for using an endoscope driving assembly for supporting and driving an endoscopic device disposed on a slave device, wherein the endoscope driving assembly comprising:
a coupler configured to support the endoscopic device; and
a mount on which the coupler is mounted and configured to receive a signal from a master device to control the endoscopic device,
wherein the coupler is replaceably provided to support a plurality of endoscopic devices with different shapes.
